# EUROPEAN PATENT SPECIFICATION

(11) **EP 1 242 004 B1**
(45) Date of publication and mention of the grant of the patent: **08.12.2004**
(21) Application number: 00983405.2
(22) Date of filing: 21.12.2000
(51) Int. Cl.: A61F 2/06

(54) **VASCULAR STENTS**
VASKULÄRE STENTS
STENTS VASCULAIRES

(30) Priority: 21.12.1999 GB 9930229; 18.02.2000 GB 0003888
(43) Date of publication of application: 25.09.2002
(73) Proprietor: Imperial College of Science, Technology and Medicine, London SW7 2AZ (GB)
(72) Inventor: CARO, Colin, Gerald, Imperial College, London SW7 2AZ (GB); DOORLY, Denis, Joseph, Imperial College, London SW7 2AZ (GB)
(74) Representative: Piésold, Alexander J.
(86) International application number: PCT/GB2000/004946
(87) International publication number: WO 2001/045593

(56) References cited:
- EP-A- 0 378 151
- EP-A- 0 791 341
- EP-A- 0 900 551
- WO-A-97/21399
- WO-A-97/37615
- DE-A- 19 634 241
- US-A- 5 104 404
- US-A- 5 772 668

## Description

This invention is concerned with vascular stents. More particularly it is concerned with vascular stents mainly for arteries although to a lesser extent for veins and other tubular vessels within the body, which stents incorporate structural features improving flow characteristics in the immediate vicinity of the surface of the stent.

Vascular stents are widely, and increasingly, used to restore flow in obstructed arteries. Many stent designs have been proposed and several are in practical use. However there are apparently technical problems with the currently available commercial stents. In particular, existing designs of stent encounter a significantly high rate of loss of patency due to thrombosis or the development of intimal hyperplasia. Whilst the exact sites at which thrombosis and intimal hyperplasia develop is a matter of some conjecture, the vascular diseases could develop at the upstream and downstream ends of stents and also within their bodies.

The most widely available stents are constructed in a hollow tubular form to a lattice pattern. Periodic discontinuities between succeeding 'zig-zag' rings of the lattice, confer the required flexibility of the stent and simultaneously ensure that the stent is sufficiently strong structurally and finely latticed to maintain the diseased artery in an open condition and prevent penetration of wall tissue into the artery or other vessel lumen.

Some of the commercially available stents presently available are constructed in this hollow tubular lattice from a shape-memory metal alloy, such as nitinol. These are frequently deployed in a collapsed state on a balloon catheter. Inflation of the balloon expands the stent when positioned in the required location.

A stent design is known which consists substantially of a single continuous wire formed as a single helix. However such stents have, in practice, been found to be insufficiently strong to be effective. The known form of single helical stent may represent the simplest form of continuous single wire stent. "Deadwater" regions in the vicinity of the stent surface are undesirable. We propose that one way of reducing the overall residence time of fluid and particles in such regions is to provide a lateral flow component along the obstacle which is usually part of the lattice i.e. the links or joining segments structure. However most commercially available stents include some form of pattern, featuring intersections. Intersections between e.g. adjacent parts of the stent according to our findings tend to produce a local fluid stagnation i.e. high residence times for fluid and particles in this immediate vicinity at the surface of the stent. We suggest that the intersections which are generally relatively rigid linear joining segments between adjacent parts of the stent, impede or block the flow at the surface vicinity of the stent and appear to represent the major cause of the local stagnation and said "deadwater" regions.

The present invention has arisen from our proposition that it is these intersections and the fact that the intersections exist within the discontinuous surface region of the stent that disrupts what should ideally be smooth and continuous flow of fluid i.e. blood in the vicinity of the said surface of the stent.

The reader will be further enlightened as to the state of the art by reference to the publications EP 0378151 A, US 5104404 and US 5772668. The present invention is particularly characterised with reference to EP 0378151.

It is from this consideration of the existing designs of commercially available stents and their perceived blood flow characteristics, that has led to the present invention.

Accordingly the present invention provides a vascular stent according to claim 1.

It is preferred for the vascular stent according to the invention to incorporate a lattice pattern of generally helical geometry. Such helical pattern, can be expected to reduce the residence time of fluid and particles within the lattice, at the lattice surface. In order that the invention may be illustrated, more easily appreciated, and readily carried into effect by those skilled in the art, embodiments of the invention will now be described by way of non-limiting example only, with reference to the accompanying drawings and in which:
Figure 1 is a schematic representation of a continuous wire single helical linked stent,
Figure 2 is an enlargement of one of the joining segments depicted in figure 1,
Figure 3 is an alternative embodiment of a single helical linked stent, of variable pitch,
Figure 4 is an embodiment of a linked single helical stent in which the single continuous wire of the helix is shaped to a space-filling curve,
Figure 5 is an embodiment of a linked double helical stent,
Figure 6 is a schematic representation of an alternative linked double helical stent arrangement with both helixes incorporating space-filling curvature,
Figure 7 is an embodiment of a stent incorporating a plurality of ring-like members as opposed to a continuous wire helical structure, and wherein external joining segments link a plurality of adjacent ring-members,
Figure 8 is an alternative linked-ring arrangement of stent in which the link between rings promotes a spiral particle migration,
Figure 9 is a view of part of surface mesh used in CFD simulation of flow in helical channel in a straight tube simulating a vascular stent, and
Figure 10 shows an array of arrows indicating magnitude and direction of cross flow velocity, particularly noteworthy is the near zero length in core but appreciable swirl component of flow near the periphery.

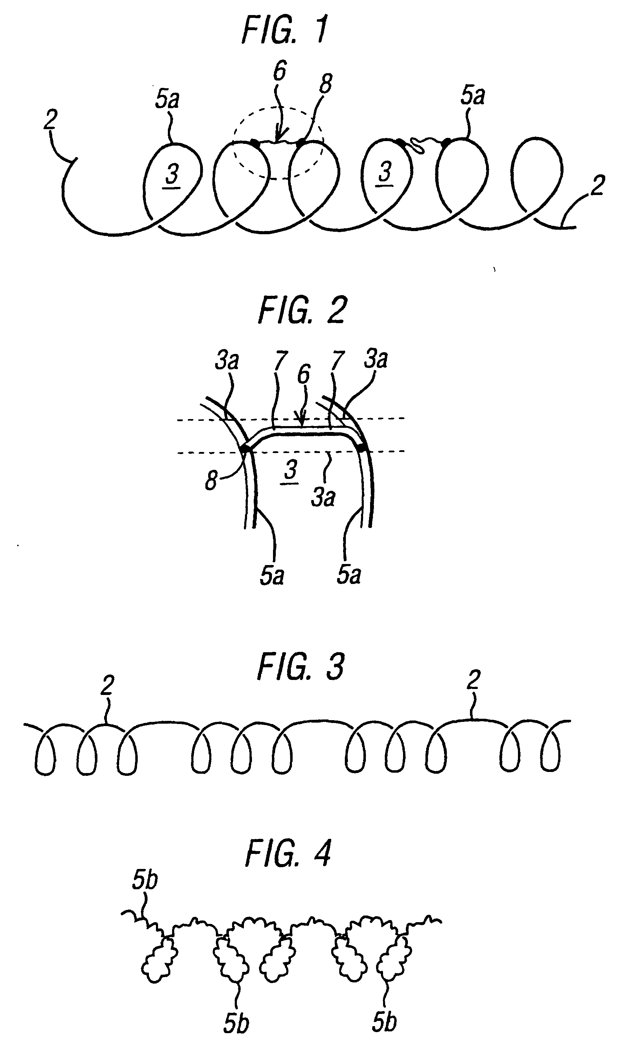

Referring to the drawings and firstly figure 1, the vascular stent 1 shown comprises a body 2 of resiliently flexible material e.g. a single continuous wire of shape memory alloy - nitinol. The body 2 defines a generally hollow tubular structure 3 the external surface 3a of which is discontinuous in that (in the unflexed condition) there are spaces between adjacent loops of the helical spiral. The discontinuous external surface corresponds to a notional cylindrical or tubular surface at the exterior of the stent, in this particular case a notional cylindrical form. In this particular embodiment, adjacent parts i.e. loops 5a of the spiral helix are spaced apart in the unflexed condition as shown. A number of loops, for example immediately adjacent loops, have been linked together by joining segments 6. The joining segment is in the nature of an elongate strip of wire which may be linear, curved or curvilinear. The ends of each joining segment 6 are secured to outermost surface parts of the single continuous wire helix 3. The joining segments similarly extend away from the notional cylindrical external surface of the single continuous wire helix and similarly a major part 7 of the joining segments is located spaced away from the said notional cylindrical surface of the stent body 2.

A plurality of similar or identical joining segments 6 can be present along the length of the body 2 of the stent. The joining segments can themselves be resiliently flexible and may be made of the same material e.g. wire as the body of the stent.

Figure 2 shows an enlarged detail of figure 1 showing a joining segment 6. Here the joints e.g. spotwelds 8 are shown outermost above the surface 3a of the body of the stent i.e. the single helical wire 3. It will be appreciated that since the joining segments 6 do not protrude into or otherwise extend within the notional cylindrical surface of the body of the stent, they are much less prone to interfere with fluid and particle flow e.g. flow of blood within the artery which has been stented, between adjacent loops of the helical spiral. The material for each joining segment 6 can also be resiliently flexible nitinol wire.

Figure 3 depicts an alternative arrangement in which the pitch of the body 2 of the single helical spiral 3 is varied. In this embodiment joining segments are present even though not illustrated.

Referring to figure 4 an alternative embodiment is shown in which the loops of the helical spiral have been twisted into a tortuous curve 5b e.g. a space-filling curve to increase the surface area of the stent wire which will be in contact with the vessel wall, after insertion. Again although not shown, in this embodiment joining segments as defined are also included.

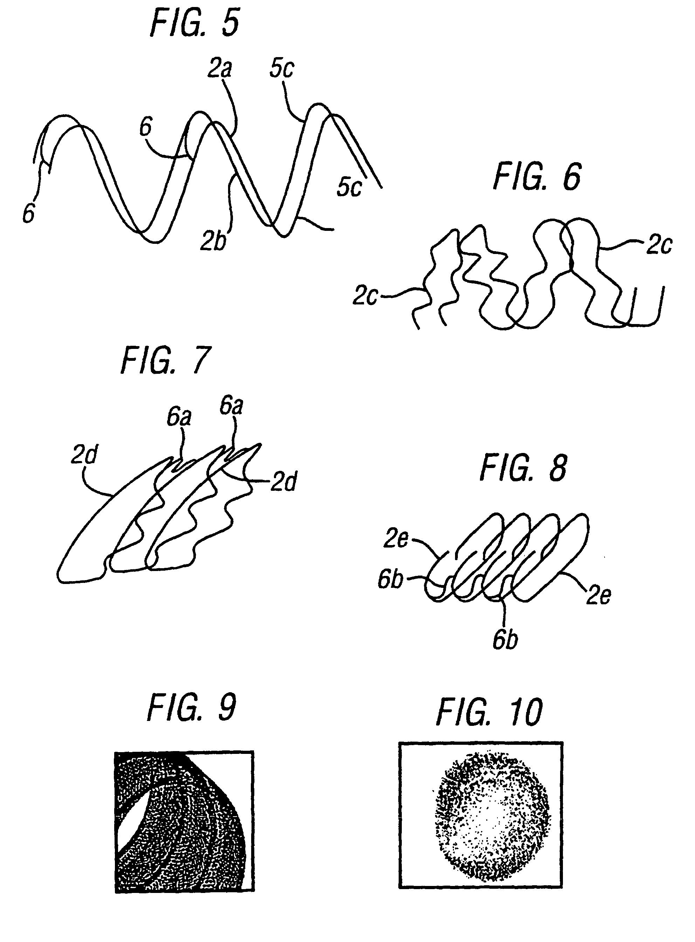

A still further alternative arrangement is proposed and this is shown in figure 5. Here, in place of a single helical spiral, a plurality of helical spirals 2a, 2b are incorporated which are linked as above, by the said joining segments 6. The spirals can be fashioned as a double helix in which adjacent loops 5c of the two respective helical spirals do not touch apart from the connection via joining segments 6.

in the arrangement of figure 6, a plurality of helical spiral configuration is shown but wherein both spirals 2c, being of single continuous wire form, have been twisted into space-filling tortuous curves. Whilst joining segments are not shown they are nevertheless incorporated within this embodiment.

In relation to double helical embodiments, it is proposed that the respective ends of these spirals (not shown) are connected in an appropriate manner to facilitate insertion of the stent form into the artery whilst simultaneously providing sufficient resilient flexibility to expand into an appropriate open hollow generally tubular configuration after insertion.

In the arrangement of Figure 7 depicted, the stent comprises a plurality of spaced apart rings 2d incorporating a curved surface and a space filling curve as shown. Adjacent parts i.e. ring members are linked by appropriately formed joining segments 6a in the form of cross-links between adjacent rings of resiliently flexible material. A major part of each crosslinking joining segment is spaced apart from the notional cylindrical surface which would be described if the outer periphery of the specially shaped ring members were continuous.

In the multiple ring-like structure of figure 7, each ring is tilted to form an oblique angle with the centre line of the stent. Though not strictly helical, the alignment of the rings shown promotes migration of flow and particles near the stent surface along the periphery of each ring-like member until the flow and particles reach the join at the top, where the links 6a are placed. The flow and particles will thus follow a path corresponding approximately to a quarter turn of a helix in each ring.

A still further arrangement is apparent from figure 8. This is essentially a linked-rin 2c arrangement in which the link 6b between rings 2c is so fashioned to promote spiral particle migration. Again the links 6b extending at the outer notional periphery of the stent have a major part spaced away from the outermost notional external surface.

Embodiments of the invention preferably establish and/or promote a swirling flow o fluid and particles in a peripheral channel of the stent e.g. as in a continuous helical chann between the individual lattice members of a hollow lattice stent according to the invention.

In preferred embodiments of the invention the predominant component of flow is along the axis of the stent, but there will be secondary flows, for example within any bends in the body of the stent.

Preferred embodiments are also provided which incorporate a continually advancin helix to maintain a favourable pressure gradient, which helps to maintain flow.

### Example 1

Steady flow in a tube with helical internal ridging/channelling, with reference to Figures 9 and 10.

Figure 9 shows a view of part of surface mesh used in CFD simulation of flow in helical channel in straight tube. Figure 10 depicts arrows showing magnitude and direction of cross flow velocity. Note near zero length in core, but appreciable swirl component near periphery.

Among observations which encourage study of the flow in a tube with helical internal ridging/channelling are: the influence of the local flow field (including wall shear stress and fluid/particle residence times) on vascular biology and pathology; the non-planar curvature and branching of arteries and associated swirling flow; and the helical distribution of atherosclerotic lesions in arteries.

We have in this example visualised the flow associated with a coiled spring (wire diameter 0.85 mm, length 5 cm, pitch in different studies 3 or 6 mm) fitted closely into a 40 cm straight length of 8 mm id PVC tubing, near its downstream end. Experiments were performed at water flow rates of 0.5, 1.0 and 6 ml/sec, representing tube Reynolds numbers (Re_{tube}) of 80, 160 and 960, respectively. Indicator (2% methylene blue) was injected through a 0.5 mm od needle at a mean velocity of approximately 0.4 mm/sec, both close to the wall between the coils and into the core flow.

| Coil Pitch 3 mm | |
|---|---|
| Re_{tube} | Channel Flow |
| 80 | helical no swirl |
| 160 | helical with swirl, swirl pitch - 2 mm |
| 960 | helical with swirl, swirl pitch - 0.5 mm |

| Coil Pitch 6 mm | |
|---|---|
| Re_{tube} | Channel Flow |
| 160 | helical no swirl |
| 960 | helical with swirl, swirl pitch - 8 mm |

Core flow was laminar in all studies and non-swirling. Channel flow was laminar in all studies and followed the helical channel configuration. With the ratio of channel depth to tube diameter fixed, channel pitch and Re_{tube} determined whether channel flow swirled and swirl pitch. The observed swirl results from separation of the flow about the channel sides (where the sides correspond to adjoining turns of the spring) combined with a pressure gradient directed along the channel. Swirling can be expected to enhance mixing and increase the uniformity of channel wall shear stress.

The ridging/channelling was made annular in some studies (a series of wire rings 3 or 6 mm apart normal to the tube axis, with wire diameter and tube id again 0.85 mm and 8 mm, respectively). Studies were performed over the same range of Re_{tube}. At the higher values of Re_{tube} indicator revealed a closed recirculation zone. At the same Re_{tube} indicator cleared faster from the helical than annular channelling, particularly at higher values of Re_{tube}.

### Example 2

An 8mm diameter commercially available corrugated ring stent (presumed to be a vascular stent) was placed in a tube and a bolus injection of indicator was made upstream of it. We found that indicator had cleared more slowly from the 'cavities' formed by the corrugated rings of the stent than from the walls of the tubing upstream.

The stent should preferably have prominent torsional flexibility; we have noted that arterial curvature and branching is commonly non-planar and shown experimentally that substantial torsional flexibility is desired for a stent to fit snugly within a tube with non-planar geometry e.g. a helix.

The stent should preferably have a lattice pattern of generally helical geometry which, additionally by virtue of joining segments being located away from (external to) the external surface, so as to reduce the residence time of fluid and particles within the lattice at the (inner) lattice surface.

## Claims

1. A vascular stent comprising a body of resiliently flexible material defining a tubular structure with a discontinuous extemal surface, in which adjacent parts of the body are spaced in an axial direction when the stent is in an unflexed condition,
a plurality of said axially spaced parts of the body being linked together by joining segments comprising a joint with the body part and a major part extending from the joint,
at least some of said joining segments being present along the length of the body and **characterised in that**
each end of each said joining segment is secured only to an outermost part of a body part and **in that** the major part of said joining segment extending between the ends is displaced away from said discontinuous external surface of the stent, so that they are less prone to interfere with fluid and particle flow.

2. A stent according to claim 1 wherein the joining segment does not protrude into a notional cylindrical surface of the stent.

3. A stent as claimed in claim 1 or claim 2 in which the adjacent parts (5a) of the body joined by a joining segment (6) are provided at least one each on adjacent turns of a generally helical spiral which promotes spiral particle migration to deter stagnation.

4. A stent as claimed in claim 1 or claim 2 in which adjacent parts (5a) of the body are discrete members.

5. A stent according to any one of the preceding claims wherein parts of the body and/or parts of the joining segments incorporate space filling curvature.

6. A stent as claimed in claim 1, 2, 3, or 5 wherein the body is in the form of a double helix said joining segments (6) link said double helixes together.

7. A stent as claimed in claim 3 wherein each discrete member is a ring tilted to form an oblique angle with a centre line of the stent.

## Patentansprüche

1. Ein Stent, der einen Körper aus elastischem, flexiblem Material umfasst, der eine röhrenförmige Struktur mit einer diskontinuierlichen äußeren Fläche definiert, bei dem benachbarte Teile des Körpers in einer axialen Richtung beabstandet sind, wenn der Stent in einer nicht gebeugten Position ist, wobei
eine Mehrzahl der axial beabstandeten Teile des Körpers durch Verbindungssegmente, die einen Anschluss mit dem Körperteil und einen Hauptteil, der sich von dem Anschluss weg ausdehnt, umfassen, verbunden sind,
mindestens einige der Verbindungssegmente entlang der Länge des Körpers vorhanden sind, **dadurch gekennzeichnet, dass**
jedes Ende von jedem der Verbindungssegmente nur an einem äußersten Teil eines Körperteils befestigt ist und dass der Hauptteil des Verbindungssegments, der sich zwischen den Enden erstreckt, von der diskontinuierlichen äußeren Fläche des Stents wegverschoben ist, so dass sie weniger geneigt sind, Flüssigkeits- und Partikelströmung zu beeinträchtigen.

2. Ein Stent nach Anspruch 1, wobei das Verbindungssegment nicht in eine theoretisch angenommene zylindrische Oberfläche des Stents hineinragt.

3. Ein Stent nach Anspruch 1 oder 2, bei dem von den benachbarten Teile (5a) des Körpers, die durch ein Verbindungssegment (6) verbunden sind; zumindest jeweils einer auf benachbarten Windungen von einer im wesentlichen spiralförmigen Schnecke, die spiralförmige Teilchenwanderung begünstigt, vorgesehen ist, um eine Stagnation zu verhindern.

4. Ein Stent nach Anspruch 1 oder 2, bei dem benachbarte Teile (5a) des Körpers separate Elemente sind.

5. Ein Stent nach einem der vorhergehenden Ansprüche, bei dem Teile des Körpers und/oder Teile der Verbindungssegmente raumfüllende Krümmungen umfassen.

6. Ein Stent nach einem der Ansprüche 1, 2, 3 oder 5, wobei der Körper die Form einer Doppelhelix bzw. -spirale hat und die Verbindungssegmente (6) die Doppelspiralen verbinden.

7. Ein Stent nach Anspruch 3, wobei jedes separate Element ein Ring ist, der schräggestellt ist, um einen schiefen Winkel mit einer Zentrallinie des Stents zu bilden.

## Revendications

1. Endoprothèse vasculaire, comprenant un corps d'un matériau souple de façon élastique, définissant une structure tubulaire avec une surface externe discontinue, dans laquelle des parties adjacentes du corps sont espacées dans une direction axiale lorsque l'endoprothèse se trouve dans un état non fléchi,
une pluralité desdites parties du corps, espacées axialement, étant reliée ensemble par des segments de jonction comprenant une jointure avec la partie de corps et une partie principale s'étendant à partir de la jointure,
au moins certains desdits segments de jonction étant présents suivant la longueur du corps et **caractérisés en ce que**
chaque extrémité de chaque dit segment de jonction est fixée uniquement à une partie tout à fait à l'extérieur d'une partie de corps et **en ce que** la partie principale dudit segment de jonction s'étendant entre les extrémités est éloignée de ladite surface externe discontinue de l'endoprothèse, de sorte qu'elles risquent moins d'interférer avec l'écoulement de fluide et de particules.

2. Endoprothèse selon la revendication 1, dans laquelle le segment de jonction ne fait pas saillie dans une surface cylindrique imaginaire de l'endoprothèse.

3. Endoprothèse selon la revendication 1 ou 2, dans laquelle les parties adjacentes (5a) du corps jointes par un segment de jonction (6) sont prévues au moins au nombre d'une respectivement sur des spires adjacentes d'une spirale généralement hélicoïdale ce qui favorise la migration de particules en spirale pour empêcher toute stagnation.

4. Endoprothèse selon la revendication 1 ou 2, dans laquelle les parties adjacentes (5a) du corps sont des éléments discrets.

5. Endoprothèse selon l'une quelconque des revendications précédentes, dans laquelle des parties du corps et/ou des parties des segments de jonction intègrent une courbure de remplissage d'espace.

6. Endoprothèse selon l'une quelconque des revendications précédentes 1, 2, 3 ou 5, dans laquelle le corps se présente sous la forme d'une hélice double, lesdits segments de jonction (6) reliant ensemble lesdites hélices doubles.

7. Endoprothèse selon la revendication 3, dans laquelle chaque élément discret est un anneau incliné pour former un angle oblique avec la ligne médiane de l'endoprothèse.
